(19) Europäisches Patentamt
European Patent Office
Office européen des brevets

(11) **EP 1 938 866 B1**

(12) **EUROPEAN PATENT SPECIFICATION**

(45) Date of publication and mention
of the grant of the patent:
**21.09.2011 Bulletin 2011/38**

(51) Int Cl.:
*A61Q 19/00* (2006.01)    *A61K 8/98* (2006.01)
*A61K 35/60* (2006.01)    *A61Q 19/02* (2006.01)

(21) Application number: **07024856.2**

(22) Date of filing: **20.12.2007**

(54) **Cosmetic product comprising a component extracted from a Salmonidae fish ovarian membrane**

Kosmetikprodukt enthaltend ein Bestandteil aus  Salmonidae-Eierstockmembrane

Produit cosmétique comprenant un produit extrait de membrane ovarienne de Salmonidae

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **22.12.2006 JP 2006346553
22.12.2006 JP 2006346552
06.09.2007 JP 2007231605
06.09.2007 JP 2007231606**

(43) Date of publication of application:
**02.07.2008 Bulletin 2008/27**

(73) Proprietor: **Nippon Barrier Free Co. Ltd.
Chiyoda-ku
Tokyo 101-0051 (JP)**

(72) Inventor: **Eto, Tadashi
Ota-ku
Tokyo 146-0085 (JP)**

(74) Representative: **Böhm, Brigitte et al
Weickmann & Weickmann
Patentanwälte
Postfach 86 08 20
81635 München (DE)**

(56) References cited:
FR-A- 2 096 704       FR-A- 2 767 697
JP-A- 57 074 062      JP-A- 2002 136 274
JP-A- 2004 073 186    JP-B1- 3 899 116
JP-B1- 3 946 238      JP-B1- 3 946 239
US-A1- 2005 271 751

**Description**

BACKGROUND OF THE INVENTION

Field of the Invention

The present invention relates to a product for inhibiting skin allergy symptoms; a cosmetic product for improving skin conditions for the lightening of pigmentation and freckles, the reduction of skin dullness, and the whitening of skin; a cosmetic product for improving darkened pores.

Description of the Related Art

A method for extracting amino acids and peptides from fish by preliminary treating the ovarian membrane (roe sac) with ozone water and enzymatically decomposing myofibril protein constituting the ovarian membrane is conventionally known (refer to Japanese Patent Laid-Open Publication No. 2004-73186).

It has been reported that the above amino acids and peptides can be used as physiologically active substances or food enriching preparations. More specifically, the amino acids and peptides are reported to have ACE inhibitory activities and thereby to work as an agent inhibiting an increase in blood pressure (an antihypertensive agent).

In addition, it has been reported that components obtained by treating a Pacific cod family, Gadidae, or Clupeidae fish ovarian membrane with a protease, which is an enzyme decomposing protein, are used as cosmetic preparations (refer to Japanese Patent Laid-Open Publication No. 2006-225270).

The components obtained by treating a Pacific cod family, Gadidae, or Clupeidae fish ovarian membrane with a protease are known to have an activity maintaining a balance among sex hormones (sex hormone-modifying activity) by activating and inhibiting the sex hormones. The above cosmetic preparations inhibit sebum secretion by the sex hormone-modifying activity of the component and improve pimples, eruptions, rough skin, and wrinkles.

Furthermore, it is known that an extract obtained by treating a salmon ovarian membrane with a protease is used as a second derivative of photoplethysmogram aging index-increasing agent, IGF-1 value-increasing agent, or rough skin-improving agent (refer to Japanese Patent Nos. 3946239, 3946238, and 3899116).

However, the extracts obtained from a fish ovarian membrane are not known for activities other than the blood pressure-decreasing activity and the sex hormone-modifying activity. Even if the extract is limited to that obtained from a salmon ovarian membrane, activities other than the second derivative of photoplethysmogram aging index-increasing activity, the IGF-1 value-increasing activity, and rough skin-improving activity are not known. Accordingly, the extracts obtained from The Salmonidae fish ovarian membrane are expected to be further developed for many applications.

FR2096704 discloses a cosmetic product comprising at least one substance derived from salmon roe and used for skin regeneration/nourishment and for maintaining the pigmentary equilibrium of the skin.

US2005/0271751 discloses cosmetic compositions based on extracts of Salmonidae fish roe for increasing the concentration of the growth factor TGFb-1 in the dermis, thus promoting the synthesis of collagen and elastin in the skin.

FR2767697 discloses dermatological compositions comprising extracts of salmonidae fish roe used as immunomodulating agents for inhibiting inflammation and allergic symptoms

SUMMARY OF THE INVENTION

Accordingly, it is an object of the present invention under the above circumstances to provide a novel application of a Salmonidae fish ovarian membrane itself or a component extracted from the ovarian membrane.

In order to achieve the object, a cosmetic product according to the present invention contains a Salmonidae fish ovarian membrane itself or a component obtained by treating the ovarian membrane with a protease.

There are known 11 genera and 211 species of fishes belonging to Salmonidae, and examples thereof include Oncorhynchus keta, Oncorhynchus nerka, Oncorhynchus salmon, Oncorhynchus mykiss, Oncorhynchus masou, and Oncorhynchus tschawytscha (King salmon). The ovarian membrane of the above Salmonidae fishes is roe sacs of the fishes belonging to Salmonidae and can be obtained by collecting roe from ovaries and washing only the sacs with water.

Furthermore, any protease that can decompose protein constituting a salmon ovarian membrane can be used.

The of the present invention can be used for inhibiting skin allergy symptoms. The product according to the present invention has an activity for inhibiting release of histamine and thereby can inhibit allergic symptoms, such as redness and itching, caused by histamine.

A cosmetic product of the present invention can be used for improving conditions of skin due to melanin synthesis. The cosmetic product according to the present invention has an activity for inhibiting synthesis of melanin and thereby can improve skin conditions, for example, the lightening of pigmentation and freckles, the reduction of skin dullness, and the whitening of skin, due to melanin synthesis.

A cosmetic product of the present invention can be used for improving darkened pores. Pores of, for example, the cheeks, forehead, and nose of a living body may be darkened. Pores are known to be darkened by deposition of a mixture of sebum, keratin, and the like, called a keratotic plug in the pores; adherence of smudges to the keratotic plug and then oxidization of the smudges or by pigmentation caused by melanin of the skin around the pores. A cosmetic product according to the present invention has an activity for reducing pore darkening and thereby can improve the darkened pores.

Furthermore, in the cosmetic product according to the present invention, the component extracted from Salmonidae fishes by treating the ovarian membrane with a protease is grains having an average grain size in the range of 200 to 1400 nm. The grains having an average grain size in the above range can be readily absorbed from the skin and can achieve higher effectiveness when they are used as a cosmetic product.

Grains having an average grain size exceeding 1400 nm may not be readily absorbed percutaneously. Contrarily, fine grains having an average grain size smaller than 200 nm are difficult to be made.

BRIEF DESCRIPTION OF THE DRAWINGS

FIG. 1 is a graph showing the number of conspicuous darkened pores when the cosmetic product of the present invention for improving darkened pores was applied to the entire face for two weeks;

FIG. 2 is a graph showing the number of pores that were improved from the conspicuous darkened pores in FIG. 1;

DESCRIPTION OF THE PREFERRED EMBODIMENTS

The preferred embodiment of the present invention will now be described in further detail with reference to the accompanying drawings.

The cosmetic product according to the embodiment contains a Salmonidae fish ovarian membrane itself or a component obtained by treating the ovarian membrane with a protease.

Examples of the fishes belonging to Salmonidae include Oncorhynchus keta, Oncorhynchus nerka, Oncorhynchus salmon, Oncorhynchus mykiss, Oncorhynchus masou, and Oncorhynchus tschawytscha (King salmon). The Salmonidae fish ovarian membrane is roe sacs of the fishes belonging to Salmonidae and can be obtained by collecting roe from the ovaries and washing only the sacs with water. In the cosmetic product according to this embodiment, the washed ovarian membrane of the Salmonidae fishes is pulverized using, for example, a mixer, a mortar, and an emulsifier and the pulverized ovarian membrane may be directly used. Alternatively, a component obtained by treating the ovarian membrane with a protease may be used.

The component obtained by treating ovarian membrane with a protease (hereinafter, the component is referred to as an ovarian membrane extraction component) can be prepared, for example, as follows:

First, a Salmonidae fish ovarian membrane is prepared as in above and is used as a raw material. To the ovarian membrane, water is added at a weight ratio of ovarian membrane : water = 1:1 to 1:3. The mixture was stirred for mixing, and a protease is added thereto in the range of 1 to 3 wt% to the total amount of the ovarian membrane. The resulting mixture is heated at 45 to 55°C for 30 minutes to 5 hours, preferably, for 2 hours. With this process, a component decomposed by the protease among the components of the ovarian membrane is eluted in the water to give an aqueous solution of the component.

Then, the protease in the above aqueous solution is inactivated. The inactivation can be conducted, for example, by heating the aqueous solution at 90°C for 5 minutes.

Then, the aqueous solution is simply filtered through wire gauze having about 30 meshes to remove coarse substances such as undecomposed ovarian membrane. Activated carbon is added to the obtained filtrate for deodorizing, decoloring, and defatting. The deodorization, decoloration, and defatting of the filtrate can be performed by adding activated carbon to the filtrate in the range of 2 to 4 wt% to the total amount of the ovarian membrane as the raw material and heating the resulting mixture at 60°C for 30 minutes, for example.

After the deodorization, the decoloration, and defatting with the activated carbon, the filtrate is filtered, for example, using a filter press. The obtained filtrate is concentrated under reduced pressure, for example, at 60°C and then is maintained, for example, at 80°C for 10 minutes for sterilization. The sterilized filtrate is dried by spray dry to give the ovarian membrane extraction component. The ovarian membrane extraction component contains amino acids, peptides, vitamins, minerals, saccharides, enzymes, nucleic acids and metabolites thereof, various growth factors, and cytokines.

The ovarian membrane extraction component may be further finely pulverized so as to have an average grain size in the range of 200 to 1400 nm. The finely pulverized ovarian membrane extraction component can be obtained by treating the sterilized filtrate with an ultra-high pressure emulsifier under a pressure in the range of 980 to 2940 MPa.

The filtrate treated with the ultra-high pressure emulsifier is dried by spray dry to give an ovarian membrane extraction component having an average grain size in the above-mentioned range.

Though reasons are not sufficiently clear, the cosmetic product of this embodiment containing the ovarian membrane itself or the ovarian membrane extraction component has an activity that can be used as any one of the following cosmetic products:

(1) a cosmetic product for inhibiting skin allergy symptoms;
(2) a cosmetic product for improving skin conditions for the lightening of pigmentation and freckles, the reduction of skin dullness, and the whitening of skin;
(3) a cosmetic product for improving darkened pores.

The cosmetic product of this embodiment can be prepared by adding, according to need, oil, a surfactant, a vitamin preparation, an ultraviolet absorber, a thickener, a humectant, an assistant material, a preservative, and a coloring and the like to the Salmonidae fish ovarian membrane itself or the component obtained by treating the ovarian membrane with a protease according to a common method. The cosmetic product may be in any form such as solution, cream, paste, gel, jelly, bubbles, a solid, granules, or powder and the like and can be used as toner, cream, ointment, lotion, emulsion, skin pack, sheet, oil, soap, cleansing, perfume, cologne, a bath liquid, a shampoo, or a rinse.

In addition, the ovarian membrane extraction component can be orally used as an agent for improving darkened pores.

Examples and Comparative Examples of the present invention will now be described.

Example 1

Roe was collected from ovaries of Oncorhynchus keta from Hokkaido, Japan, and the ovarian membrane was sufficiently washed with water. Purified water (100 mL) was added to 100 g of the washed ovarian membrane, and the mixture was pulverized using a mixer (manufactured by Matsushita Electric Industrial Co., Ltd. under the name of MX-X107) for one minute. This pulverization process was repeated ten times to give a pulverized substance. The pulverized substance was further ground using a mortar and then filtered through wire gauze having 300 meshes, and the resultant substance was used as a sample.

The sample prepared in this Example is a pulverized substance of ovarian membrane of Oncorhynchus keta. Then, this sample prepared in this Example was subjected to a histamine-release inhibition test.

In the histamine-release inhibition test, the above sample was diluted with purified water to prepare a sample solution containing 0.2% of solid contents.

Then, 0.2 mL of the sample solution and a histamine-releasing agent (available from Sigma-Aldrich under the name of Compound 48/80) were added to 1.2 mL of a suspension of mast cells isolated from the peritoneal cavity of rats (Slc:Wister male rat, about 4 to 9 weeks old) to prepare a mixture solution containing the sample at a concentration of 1 $\mu$g/mL.

The resulting mixture solution was cultured at 37°C for 15 minutes, and the reaction was terminated by cooling the solution with ice. The reaction solution was subjected to centrifugation. The released histamine was collected from the supernatant and purified. Then, the purified histamine was reacted with o-phthaldialdehyde and the fluorescence absorbance was measured with an excitation wavelength of 360 nm and a fluorescence wavelength of 450 nm. The histamine-release inhibition rate was calculated according to the following equation:

$$\texttt{Histamine-release inhibition rate (\%) = \{1-(A-C)/(B-C)\} \times 100}$$

In the equation, A represents the fluorescence intensity of histamine released from mast cells in the presence of the sample and the histamine-releasing agent; B represents the fluorescence intensity of histamine released from mast cells in the presence of the histamine-releasing agent; and C represents the fluorescence intensity of histamine naturally released from mast cells. Each of these A, B, and C values is obtained by subtracting a blind test value from the measurement value.

The results are shown in Table 1.

Example 2

300 mL of purified water was added to the sample prepared in Example 1, and the mixture was pulverized using a high pressure emulsifier (manufactured by Mizuho Industrial Co., Ltd. under the name of Microfluidizer M110-E/H) at a treatment pressure of 150 MPa. This pulverization process was repeated twice to give a sample.

The sample prepared in this Example is a finely pulverized substance of ovarian membrane of Oncorhynchus keta. Then, except for the fact that the sample prepared in this Example was used, the same histamine-release inhibition test as in Example 1 was carried out to determine the histamine-release inhibition rate. The results are shown in Table 1.

Example 3

The sample (aqueous solution) prepared in Example 2 was adjusted to a pH of 7.2 to 7.5, and 0.5 g of

Actinase E (manufactured by Kaken Pharmaceutical Co., Ltd.) was added thereto as a protease. The resulting mixture was stirred at 40°C for 3 hours for the reaction.

Then, the protease in the aqueous solution was inactivated by heating the solution at 90°C for 5 minutes, and then the aqueous solution was simply filtered through a filter having 30 meshes to remove coarse substances such as undecomposed ovarian membrane.

Then, 500 g of activated carbon was added to the filtrate obtained by the simple filtration. The mixture was heated at 60°C for 30 minutes for defatting, decoloration, and deodorization of the filtrate. The filtrate was filtered using a filter press, and the obtained filtrate was concentrated under reduced pressure and sterilized. The sterilized filtrate was dried by spray dry to give a sample.

The sample prepared in this Example is an ovarian membrane extraction component of Oncorhynchus keta. Then, except for the fact that the sample prepared in this Example was used, the same histamine-release inhibition test as in Example 1 was carried out to determine the histamine-release inhibition rate. The results are shown in Table 1.

[Table 1]

|  | Histamine-release inhibition rate (%) |
|---|---|
| Example 1 | 77.9 |
| Example 2 | 69.2 |
| Example 3 | 69.9 |

As shown in Table 1, it is obvious that both The Salmonidae fish ovarian membrane prepared in Examples 1 and 2 and the ovarian membrane extraction component obtained by treating a Salmonidae fish ovarian membrane with a protease in Example 3 have excellent histamine-release inhibitory activities. Therefore, it is obvious that a cosmetic product having a histamine-release inhibitory activity can be obtained by using The Salmonidae fish ovarian membrane or the ovarian membrane extraction component of Salmonidae fishes. Such a cosmetic product can be expected to have an effect inhibiting allergic symptoms, such as redness and itching, caused by histamine.

Example 4

Roe was collected from ovaries of Oncorhynchus keta from Hokkaido, Japan, and the ovarian membrane was sufficiently washed with water. Purified water (100 mL) was added to 100 g of the washed ovarian membrane, and the mixture was pulverized using a mixer (manufactured by Matsushita Electric Industrial Co., Ltd. under the name of MX-X107) for one minute. This pulverization process was repeated ten times to give a pulverized substance. The pulverized substance was further ground using a mortar and then filtered through wire gauze having 300 meshes, and the resultant substance was used as a sample.

The sample prepared in this Example is a pulverized substance of ovarian membrane of Oncorhynchus keta. Then, this sample prepared in this Example was subjected to a melanin synthesis inhibition test.

In the melanin synthesis inhibition test, the above sample was diluted with an MEM culture medium containing 10% FBS (hereinafter referred to as "10% FBS-containing MEM culture medium") to prepare test solution A containing 100 ppm of the sample and test solution B containing 300 ppm of the sample.

Then, B-16 mouse melanoma cells, which synthesize melanin, were cultured in the 10% FBS-containing MEM culture medium. A cell suspension containing $1 \times 10^5$ cells/mL of the B-16 mouse melanoma cells was prepared using the culture medium. 5 mL of the cell suspension were seeded into each of ten 25-mL flasks. The B-16 mouse melanoma cells seeded in each flask were cultured at 37°C for 24 hours in an incubator with $CO_2$ concentration controlled at 5%.

The B-16 mouse melanoma cells were confirmed to be completely adhered to each flask, and then the 10% FBS-containing MEM culture medium was removed from each flask. Five milliliters of the test solution A was added to three of the ten flasks, 5 mL of the test solution B was added to another three of the flasks, and 5 mL of the 10% FBS-containing MEM culture medium was added to another three of the flasks as control. The cells in these flasks were cultured for six days. On the third day after the start of the culturing, the test solutions A and B and the 10% FBS-containing MEM culture medium were exchanged with 5 mL of fresh test solution A and B and the 10% FBS-containing MEM culture medium, respectively.

After the completion of the culturing, the B-16 mouse melanoma cells were treated with trypsin and collected.

The collected B-16 mouse melanoma cells were observed for degree of whiteness by a naked eye. The observation results are shown in Table 2. In Table 2, "±" represents a whiteness degree substantially the same as that of the control, and "+", "++", and "+++" each represents a higher whiteness degree with the increase in the number of "+".

Example 5

300 mL of purified water was added to the sample prepared in Example 4, and the mixture was pulverized using a high pressure emulsifier (manufactured by Mizuho Industrial Co., Ltd. under the name of Microfluidizer M110-E/H) at a treatment pressure of 150 MPa. This pulverization process was repeated twice to give a sample.

The sample prepared in this Example is a finely pulverized substance of ovarian membrane of Oncorhynchus keta. Then, except for the fact that the sample prepared in this Example was used, the same melanin synthesis inhibition test as in Example 4 was carried out. The results are shown in Table 2.

Example 6

The sample (aqueous solution) prepared in Example 5 was adjusted to a pH of 7.2 to 7.5, and 0.5 g of Actinase E (manufactured by Kaken Pharmaceutical Co., Ltd.) was added thereto as a protease. The resulting mixture was stirred at 40°C for 3 hours for the reaction.

Then, the protease in the aqueous solution was inactivated by heating the solution at 90°C for 5 minutes, and then the aqueous solution was simply filtered through a filter having 30 meshes to remove coarse substances such as undecomposed ovarian membrane.

Then, 500 g of activated carbon was added to the filtrate obtained by the simple filtration. The mixture was heated at 60°C for 30 minutes for defatting, decoloration, and deodorization of the filtrate. The filtrate was filtered using a filter press, and the obtained filtrate was concentrated under reduced pressure and sterilized. The sterilized filtrate was dried by spray dry to give a sample.

The sample prepared in this Example is an ovarian membrane extraction component of Oncorhynchus keta. Then, except for the fact that the sample prepared in this Example was used, the same melanin synthesis inhibition test as in Example 4 was carried out. The results are shown in Table 2.

[Table 2]

| | | Whiteness degree |
|---|---|---|
| Example 4 | Test solution A | ++ |
| | Test solution B | +++ |
| Example 5 | Test solution A | +++ |
| | Test solution B | +++ |
| Example 6 | Test solution A | ++ |
| | Test solution B | +++ |

As shown in Table 2, it is obvious that both the Salmonidae fish ovarian membrane prepared in Examples 4 and 5 and the ovarian membrane extraction component obtained by treating a Salmonidae fish ovarian membrane with a protease in Example 6 have excellent melanin synthesis inhibitory activities. Therefore, it is obvious that a cosmetic product having a melanin synthesis inhibitory activity can be obtained by using the Salmonidae fish ovarian membrane or the ovarian membrane extraction component of Salmonidae fishes. Such a cosmetic product can be expected to have an effect improving skin conditions for the lightening of pigmentation and freckles of skin, the reduction of skin dullness, and the whitening of skin.

Example 7

Roe was collected from ovaries of Oncorhynchus keta from Hokkaido, Japan, and the ovarian membrane was sufficiently washed with water. Purified water (100 mL) was added to 100 g of the washed ovarian membrane, and the mixture was pulverized using a mixer (manufactured by Matsushita Electric Industrial Co., Ltd. under the name of MX-X107) for one minute. This pulverization process was repeated ten times to give a pulverized substance. The pulverized substance was further ground using a mortar and then filtered through wire gauze having 300 meshes.

To the resultant substance, 300 mL of purified water was added. The mixture was pulverized using an ultra-high pressure emulsifier (manufactured by Mizuho Industrial Co., Ltd. under the name of Microfluidizer M110-E/H) at a treatment pressure of 150 MPa. This pulverization process was repeated twice to give an aqueous solution of finely pulverized substance of ovarian membrane of Oncorhynchus keta.

The aqueous solution was adjusted to a pH of 7.2 to 7.5, and 0.5 g of Actinase E (manufactured by Kaken Pharmaceutical Co., Ltd.) was added thereto as a protease. The resulting mixture was stirred at 40°C for 3 hours for the reaction.

Then, the protease in the aqueous solution was inactivated by heating the solution at 90°C for 5 minutes, and then the aqueous solution was simply filtered through a filter having 30 meshes to remove coarse substances such as undecomposed ovarian membrane.

Then, 500 g of activated carbon was added to the filtrate obtained by the simple filtration. The mixture was heated at 60°C for 30 minutes for defatting, decoloration, and deodorization of the filtrate. The filtrate was filtered using a filter press, and the obtained filtrate was concentrated under reduced pressure and sterilized. The sterilized filtrate was concentrated under reduced pressure, for example, at 60° C to give an ovarian membrane extraction component of Oncorhynchus keta as a filtrate.

The above concentrated filtrate was treated with the above ultra-high pressure emulsifier under a pressure in the range of 980 to 2940 MPa for allowing the ovarian membrane extraction component of Oncorhynchus keta to become grains having an average grain size in the range of 200 to 1400 nm. The filtrate treated with the ultra-high pressure emulsifier was dried by spray dry to give a grained salmon ovarian membrane extraction component (Sample A).

Then, a lotion for improving darkened pores was prepared using the above Sample A so as to have the composition shown in Table 3 according to a common method. Similarly, an emulsion for improving darkened pores was prepared using the above Sample A so as to have the composition shown in Table 4 according to a common method.

[Table 3]

| Component | Weight (g) |
|---|---|
| Sample A | 0.5 |
| Purified water | 89.781 |
| 1,3-Butylene glycol | 3.0 |
| Ethanol | 4.0 |
| Glycerin | 2.0 |
| Methyl paraoxybenzoate | 0.1 |
| Polyoxyethylene hydrogenated castor oil | 0.1 |
| Natural vitamin E | 0.019 |
| Sodium hyaluronate | 0.5 |
| Total | 100.0 |

[Table 4]

| Component | Weight (g) |
|---|---|
| Sample A | 0.5 |
| Purified water | 92.375 |
| 1,3-Butylene glycol | 2.0 |
| Glycerin | 3.0 |
| 1,2-Pentanediol | 2.0 |
| Methyl paraoxybenzoate | 0.1 |

(continued)

| Component | Weight (g) |
|---|---|
| Xanthan gum | 0.025 |
| Total | 100.0 |

The lotion and emulsion for improving darkened pores were applied to the entire face of each of 12 monitors (38- to 42-year old healthy females) twice a day (morning and evening) after washing the face for two weeks. All monitors do not have pollen allergy and salmon allergy, do not smoke, do not take any drugs, including pills, regularly, have not received cosmetic medical care, are not pregnant, and are not in lactation.

Black and white images of the skin of the entire face were taken at the start of the application and after the application for two weeks using a skin image analyzer (manufactured by Inforward, Inc. under the name of Robo Skin Analyzer) under a uniform light source. Then, the numbers of conspicuous darkened pores in the skin images were counted. In the skin images, when the brightness of color was expressed by 0 to 255 gray-scale levels (0 representing the darkest and 255 representing the lightest), a region having a gray-scale level of 0 to 215 and an area of 0.1 to 0.6 mm$^2$ was defined as "pore". Among the regions corresponding to pores, a region containing a portion having a gray-scale level of 0 to 40 was defined as "conspicuous darkened pores".

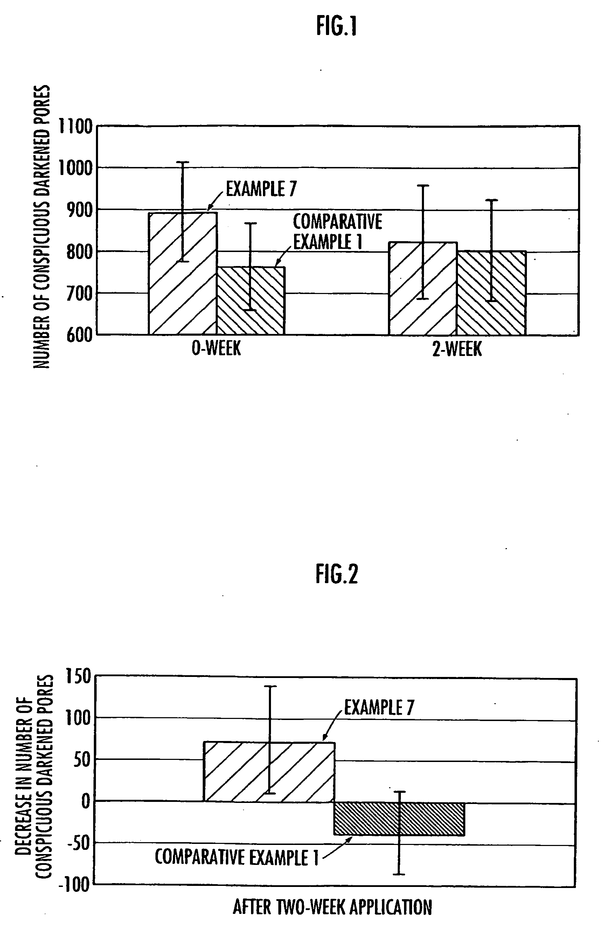

FIG. 1 shows the average value of the numbers of conspicuous darkened pores of the 12 monitors. FIG. 2 shows the number of pores that were improved in the conspicuous darkened pores in FIG. 1. In FIG. 1, the average value before the application is mentioned as "0-week", and the average value after the application for 2 weeks is mentioned as "2-week".

Comparative Example 1

In this Comparative Example, a lotion (placebo) not containing Sample A was prepared so as to have the composition shown in Table 5 according to a common method, instead of the lotion for improving darkened pores in Example 7. The placebo lotion had the same composition as that of the lotion for improving darkened pores in Example 7 other than that the same amount of purified water was used instead of Sample A.

Furthermore, a placebo emulsion not containing Sample A was prepared so as to have the composition shown in Table 6 according to a common method, instead of the emulsion for improving darkened pores in Example 7. The placebo emulsion had the same composition as that of the emulsion for improving darkened pores in Example 7 other than that the same amount of purified water was used instead of Sample A.

[Table 5]

| Component | Weight (g) |
|---|---|
| Purified water | 90.281 |
| 1,3-Butylene glycol | 3.0 |
| Ethanol | 4.0 |
| Glycerin | 2.0 |
| Methyl paraoxybenzoate | 0.1 |
| Polyoxyethylene hydrogenated castor oil | 0.1 |
| Natural vitamin E | 0.019 |
| Sodium hyaluronate | 0.5 |
| Total | 100.0 |

[Table 6]

| Component | Weight (g) |
|---|---|
| Purified water | 92.875 |
| 1,3-Butylene glycol | 2.0 |

(continued)

| Component | Weight (g) |
|---|---|
| Glycerin | 3.0 |
| 1,2-Pentanediol | 2.0 |
| Methyl paraoxybenzoate | 0.1 |
| Xanthan gum | 0.025 |
| Total | 100.0 |

The placebo lotion and emulsion were applied to the entire face of each of 12 monitors (38- to 42-year old healthy females, different from the monitors in Example 7) twice a day (morning and evening) after washing the face for two weeks. All monitors do not have pollen allergy and salmon allergy, do not smoke, do not take any drugs, including pills, regularly, have not received cosmetic medical care, are not pregnant, and are not in lactation.

The numbers of conspicuous darkened pores were counted by the same method as in the above Example. FIG. 1 shows the average value of the numbers of conspicuous darkened pores of the 12 monitors. FIG. 2 shows the number of pores that were improved in the conspicuous darkened pores in FIG. 1.

As shown in FIG. 1, it is obvious that the number of conspicuous darkened pores was decreased by the application of the lotion and emulsion for improving darkened pores according to this embodiment (Example 7) for two weeks. On the other hand, it is obvious that the number of conspicuous darkened pores was slightly increased by the application of the placebo lotion and emulsion in Comparative Example 1 for two weeks.

As shown in FIG. 2, it is obvious that the number of conspicuous darkened pores was remarkably decreased by the lotion and emulsion for improving darkened pores according to this embodiment.

Therefore, it is obvious that the lotion and emulsion for improving darkened pores according to this embodiment can alleviate and improve darkened pores by being applied to skin.

A new application of a component extracted from a Salmonidae fish ovarian membrane is provided. A cosmetic product of the present invention contains a Salmonidae fish ovarian membrane or a component obtained by treating the ovarian membrane with a protease. The cosmetic product can be used as any one of (1) a cosmetic product for inhibiting skin allergy symptoms; (2) a cosmetic product for improving skin conditions for the lightening of pigmentation and freckles, the reduction of skin dullness, and the whitening of skin; (3) a cosmetic product for improving darkened pores. In the cosmetic products for improving darkened pores, the component obtained by treating The Salmonidae fish ovarian membrane with a protease is preferably grains having an average grain size of 200 to 1400 nm.

**Claims**

1. Cosmetic, non-Therapeutic use of a cosmetic product comprising a Salmonidae fish ovarian membrane, for improving skin conditions for the lightening of pigmentation and freckles, the reduction of skin dullness, and the whitening of skin wherein the cosmetic product has a melanin synthesis inhibitory activity.

2. A product comprising a Salmonidae fish ovarian membrane, for use as a medicament for inhibiting skin allergy symptoms wherein the cosmetic product has a histamine-release inhibitory activity.

3. The use of a cosmetic product according to claim 1 wherein the cosmetic product comprises a component obtained by treating a Salmonidae fish ovarian membrane with a protease.

4. The product according to claim 2, comprising a component obtained by treating a Salmonidae fish ovarian membrane with a protease.

5. Cosmetic, non-Therapeutic use of a cosmetic product comprising a compound obtained by treating a Salmonidae fish ovarian membrane with a protease for improving darkened pores.

6. The use of a cosmetic product according to claim 5, wherein the component obtained by treating a Salmonidae fish ovarian membrane with a protease is grains having an average grain size in the range of 200 to 1400 nm.

**EP 1 938 866 B1**

**Patentansprüche**

1. Kosmetische, nicht-therapeutische Verwendung eines Kosmetikprodukts, umfassend Salmonidae Fischovarmembran, zur Verbesserung von Hautzuständen zur Aufhellung von Pigmentierung und Sommersprossen, zur Verminderung von Hautfahlheit und zum Bleichen von Haut, wobei das Kosmetikprodukt eine die Melaninsynthese hemmende Wirkung aufweist.

2. Produkt, umfassend eine Salmonidae Fischovarmembran, zur Verwendung als Medikament zur Hemmung von Hautallergiesymptomen, wobei das Produkt eine die Histaminfreisetzung hemmende Wirkung aufweist.

3. Verwendung eines Kosmetikprodukts nach Anspruch 1, wobei das Kosmetikprodukt einen Bestandteil umfasst, der durch Behandlung einer Salmonidae Fischovarmembran mit einer Protease erhalten wurde.

4. Produkt nach Anspruch 2, umfassend einen Bestandteil, der durch Behandlung einer Salmonidae Fischovarmembran mit einer Protease erhalten wurde.

5. Kosmetische, nicht-therapeutische Verwendung eines Kosmetikprodukts, welches durch Behandlung einer Salmonidae Fischovarmembran mit einer Protease erhalten wurde, zur Verbesserung dunkler Poren.

6. Verwendung eines Kosmetikprodukts nach Anspruch 5, wobei es sich bei dem Bestandteil, der durch Behandlung einer Salmonidae Fischovarmembran mit einer Protease erhalten wurde, um Körner mit einer durchschnittlichen Korngröße im Bereich von 200 bis 1400 nm handelt.

**Revendications**

1. Utilisation cosmétique, non thérapeutique d'un produit cosmétique comprenant une membrane ovarienne de poisson salmonidé, pour l'amélioration des conditions de la peau, pour l'éclaircissement de la pigmentation et des taches de rousseur, la réduction de l'aspect terne de la peau, et pour le blanchiment de la peau, dans laquelle le produit cosmétique a une activité d'inhibition de la synthèse de la mélanine.

2. Produit comprenant une membrane ovarienne de poisson salmonidé, pour utilisation en tant que médicament pour l'inhibition des symptômes d'une allergie cutanée, dans lequel le produit cosmétique a une activité d'inhibition de la libération de l'histamine.

3. Utilisation d'un produit cosmétique selon la revendication 1, dans laquelle le produit cosmétique comprend un composant obtenu par traitement d'une membrane ovarienne de poisson salmonidé avec une protéase.

4. Produit selon la revendication 2, comprenant un composant obtenu par traitement d'une membrane ovarienne de poisson salmonidé avec une protéase.

5. Utilisation cosmétique, non thérapeutique d'un produit cosmétique comprenant un composé obtenu par traitement d'une membrane ovarienne de poisson salmonidé avec une protéase pour améliorer les comédons.

6. Utilisation d'un produit cosmétique selon la revendication 5, dans laquelle le composant obtenu par traitement d'une membrane ovarienne de poisson salmonidé avec une protéase est des grains ayant une taille moyenne de grains dans la plage de 200 à 1 400 nm.

## FIG.1

## FIG.2

**EP 1 938 866 B1**

**REFERENCES CITED IN THE DESCRIPTION**

**Patent documents cited in the description**

- JP 2004073186 A
- JP 2006225270 A
- JP 3946239 B
- JP 3946238 B
- JP 3899116 B
- FR 2096704
- US 20050271751 A
- FR 2767697